# EUROPEAN PATENT APPLICATION

(11) **EP 4 129 204 A1**
(43) Date of publication of application: **08.02.2023**
(21) Application number: 21788158.0
(22) Date of filing: 18.03.2021
(51) Int. Cl.: A61B 17/02, A61M 25/10

(54) **CURVED BALLOON CATHETER RETRACTOR**

(30) Priority: 16.04.2020 CN 202010302531
(71) Applicant: Shanghai Keci Medical Technology Co., Ltd, Shanghai 201613 (CN)
(72) Inventor: ZHANG, Zhichao, Shanghai 200135 (CN); MA, Changsheng, Shanghai 200135 (CN); SUBBAKRISHNA, Shankar, Shanghai 200135 (CN)
(74) Representative: Cameron Intellectual Property Ltd
(86) International application number: PCT/CN2021/081406
(87) International publication number: WO 2021/208663

(57) **Abstract**

Disclosed is a curved balloon catheter retractor, comprising a positioning balloon (11) provided at the head of a catheter (10), and a bendable curved balloon (12) provided at a portion on the catheter (10) that is close to the head, wherein the positioning balloon (11) and the curved balloon (12) are respectively connected to respective injection ports at the tail of the catheter (10) by means of pipelines inside the catheter (10); and a handle (14) is provided at the tail of the catheter (10) for adjusting the position of the catheter (10). In a non-use state, the positioning balloon (11) and the curved balloon (12) are tightly attached to the catheter (10). In an in-use state, fluid is injected from the injection port of the positioning balloon (11), such that the positioning balloon (11) is expanded to achieve the positioning effect; and the fluid is then injected into the curved balloon (12), such that the curved balloon (12) and part of the catheter (10) in the curved balloon are curled and deflected towards one side to create the retraction. In this way, simple and reliable retraction of the catheter (10) is achieved.

## Description

### FIELD OF THE INVENTION

The present invention relates to a catheter retractor, and more particularly to a curved balloon catheter retractor.

### BACKGROUND OF THE INVENTION

The retractor is also referred to as a drag hook or retractor for retracting tissue, exposing a desired surgical range, facilitating exploration and operation, and may be divided into two categories: a hand-held pull hook and an automatic pull hook. There are various specifications and sizes of different shapes and sizes, and a suitable hook can be selected according to the operation requirements.

Conventional retractors require a larger operating space, which requires a larger surgical wound. At the same time, most of the traditional retractors are made of metals, and usually with a sharp end, which is easy to cause secondary trauma to the patient and damage important organ tissue.

Currently, retractable tissue retractors are widely used, and can be passed through the working channel of the retractable endoscope. Tissue retractors are used in endoscopic and open surgeries, including retractable endoscopic, laparoscopic, and general surgical procedures. In order to accommodate specific requirements of surgeries, the length and diameter of such a tissue retractor may be fixed or may vary. A retractable endoscope can be used to fix or secure the organ tissue, to pull it and to operate on it in some manner.

The catheter retractor is used for tissue retraction in surgery, which completes the retraction operation through natural lumen interventional surgeries or open surgeries. Surgeries include, but are not limited to, various laparoscopic surgery, cardiovascular surgery, brain surgery, gastrointestinal surgery, urinary surgery, etc. Tissue for retraction including but not limited to gastrointestinal tract, esophagus, airway, urethra, vagina, bladder, etc. The purposes of retraction include, but are not limited to, protecting a particular tissue, and/or removing a particular tissue to facilitate surgical operations.

The following example uses atrial fibrillation ablation complications to introduce the use and action of the catheter retractor. Atrial fibrillation is the most common arrhythmia, while atrial fibrillation ablation therapy has been gradually recognized in recent years as a better solution than drug therapy and conventional surgical procedures. The atrial fibrillation catheter ablation surgical therapy technology was reported for the first time in 1996, and has gradually become a clinically mature treatment technology after 20 years of technological progress and experience accumulation. The surgical success rate and the complication rate of atrial fibrillation ablation are all improved year by year, but the serious complication rate thereof is still 1% -3%. The more experienced an operator or surgeon is, the lower the probability of complications. If the atrial fibrillation ablation procedure is expected to be more commonly used in hospitals, improving the safety indicator is most important, and the following complications need to be well controlled.

Stroke (Cause: Scab and Thrombus Shedding in Intraoperative Wound), Incidence: 0.1% -0.5%. Heart perforation (Cause: mechanical trauma due to ablation), Incidence: 0.2% -0.5%. Pulmonary vein stenosis (Reason: too deep ablation or ablation site), Incidence: < 0.1%. Left Atrial Esophageal Fistula (Cause: ablation causes injury to esophagus), Incidence: 0.3-0.5%, Mortality: > 75%.

The occurrence of atrial esophageal fistula ("AEF") complications arises from the spatial relationship between the left atrium and the esophagus. Since the esophagus is behind the posterior mediastinum, the posterior wall of the left atrium is only spaced from the pericardial sinus, while the posterior wall of the left atrium and the anterior wall of the esophagus are both thin, and high energy used in the ablation procedure is likely to cause excessive damage to the esophagus. AEF complications have the following characteristics: extremely high mortality, current rescue success cases are very few, and treatment means are very limited. It has been found that the diagnosis difficulty is high, since the symptoms of AEF complications usually occur after several weeks to months post-surgery.

By reducing the lethal rate of the procedure, the failure rate of the procedure and the occurrence of doctor-patient disputes will be reduced. Some patients avoid surgery due to fear of the occurrence of AEF complications and lose the opportunity to cure atrial fibrillation. If the occurrence of esophageal fistula can be effectively prevented, many patients who have worried the risk of AEF will be willing to receive AF surgical ablation treatment. In addition, the physician surgeons usually reduce the ablation power used during surgery in order to reduce the risk of AEF complications. As a result, the radical cure rate of the ablation procedure is reduced. By preventing the occurrence of the esophageal fistula, the doctor can perform ablation work on the region near the esophagus using the normal power, thereby improving the root treatment rate of atrial fibrillation.

For the patient with AEF, the esophagus and the left atrium are usually too close (about 1 cm), therefore the energy used during the atrial fibrillation ablation surgery can easily injure the esophagus. The technology of esophagus retraction fundamentally solves this problem. It has been proved that the method has a good effect on the control of esophageal injury and the prevention of AEF complications. Therefore the safety and effectiveness of the esophageal retraction are both preliminarily verified.

In a study presented at the 2017 AF Symposium, an International Atrial Fibrillation Symposium ("2017 AF Symposium"), a study of 101 patients who had atrial fibrillation ablation with esophageal retraction showed that none of the patients' esophagus temperatures exceeded 38°C in the study. The study simultaneously recorded various side effects of the digestive tract of 101 patients, such as dysphagia, blood circulation, dyspepsia, and other gastrointestinal symptoms, and follow-up for at least 6 months. The follow-up results showed that only a few patients had dysphagia (occurrence rate of 7%), which occurs immediately after operation and is completely relieved in several days, and no gastrointestinal complications were observed afterwards.

At present, most tools for esophageal retraction used in the research are devices without bendable ends and not special made for retraction, such as tracheal probes, endoscopes, etc. These devices have clinical problems such as complex operation procedures, limited retraction deviation distance, and incapability of completely adapting to esophageal structures.

In order to make the mechanical structure generate enough rigidity to achieve a satisfactory pulling effect, the mechanical structure generally has a larger diameter, so that the mechanical structure can only be inserted into the esophagus through the mouth, and more discomfort is brought to the patient. Meanwhile, due to the large diameter, the mechanical structure is limited in application field, and cannot be applied to other narrow and structurally complex human cavities.

### SUMMARY OF THE INVENTION

The purpose of the present invention is to provide a curved balloon catheter retractor for natural lumen or cavity intervention or open surgical intervention based on the above problems, and to solve the problem that current catheter retractors are complex in structure, cannot accurately control the pulling force, and have low reliability, thereby achieving a simple and reliable catheter retraction and retractor.

In order to achieve the above object, the present invention provides a curved balloon catheter retractor. The distal end of the catheter is provided with a positioning balloon. There is a curved balloon close to the distal end. The positioning balloon and the curved balloon are connected to respective injection ports at the proximal end of the catheter through lumens inside the catheter. The proximal end of the catheter is provided with a handle for adjusting the position of the catheter. In the unused state, the positioning balloon and the curved balloon are tightly attached to the catheter; while in the use state, fluid is injected by the injection port of the positioning balloon, and the positioning balloon is inflated to achieve the positioning effect; and then the fluid is injected into the curved balloon, so that the curved balloon and the catheter portion in the curved balloon are bent or in a bended or curled position, and the retraction is achieved by deviating or shifting the tissue or organ to one side.

In the unused state, the positioning balloon and the curved balloon are tightly attached to the catheter and covered by a rigid tubular protective shield. The balloon protective shield is removed when the balloon is in use. The handle has a length scale portion for marking the depth of the catheter insertion. The catheter is made of a material that is not easily twisted, or the catheter is reinforced with a material that is not easily twisted. There are three radiopaque parts or developing devices in the curved balloon, which are used for displaying the relative positions of the proximal end, the middle part and the distal end of the curved balloon respectively. A radiopaque line is provided on the catheter for display under X-ray, for development and use. The length of the curved balloon is preferably 9-11 cm. The number of curved balloon segments is in the range of 5 to 8.

Further, the proximal end or tail portion of the catheter is provided with a main shaft for holding and rotating, and the proximal end of the main shaft is provided with a fixing screw for clamping the catheter passing through the interior of the main shaft. A head portion of the main shaft is provided with a locking sliding block for fixing or loosening the catheter passing through the interior of the main shaft for adjusting the position of the catheter. The main shaft head portion has two connecting buckles for connecting a fixing headband such that the main shaft and the entire catheter are fixed relative to the patient's head.

Further, one end of a high-pressure braided tube is further provided with a syringe, and another end of the high-pressure braided tube is connected with an injection port of the curved balloon or positioning balloon at the distal end of the catheter. A two-way valve is further provided with a switch in the middle of the two-way valve. The two-way valve can be connected between the injection port and the high-pressure braided tube when needed to control fluid injection so as to maintain the pressure within the balloon.

Preferably, the head band is further provided with a main body portion located at the back of the head, and the main body portion is provided with a large hole for accommodating a protruding portion at the back head, making it more comfortable to wear. The main body portion is provided with four connecting belts, and each connecting belt is provided with a string of connecting holes which can clamp and connect with the two connecting buckles of the head portion of the main shaft.

Preferably, a sheath tube is further provided, which is a hollow tube, and the sheath tube can form a fixed non-rotating protective portion, so that an upper portion of the natural lumen or human body cavity is not damaged by rotation of the catheter. A radiopaque line is embedded in the sheath tube, and the position and state of the sheath tube can be observed by X-ray development when entering and within the human body. The sheath tube is provided with a hole for spraying a developing solution to display the position where the curved balloon is placed or located. The bottom of the sheath tube is provided with a three-way round joint, and one end of the sheath tube is connected with the round joint, and one end of the round joint is connected with the main shaft so as to accommodate the catheter, and the side edge of the round joint is provided with a hole for injecting a developing solution.

Further, the sheath tube auxiliary device is a flexible or soft solid tube, and the solid tube has a smooth head and an oval bottom. The sheath tube auxiliary device is used to smoothly enter the human cavity by using the characteristics of the flexible solid tube, and meanwhile guide the sheath tube, so that the sheath tube is prevented from being inserted directly to the cavity in such a way as to cause the sheath tube edge to scratch the human cavity, such as the nasal mucosa or the esophageal wall. After the sheath tube is introduced into the cavity, the sheath tube auxiliary device can be pulled out smoothly. The catheter is then inserted into the sheath tube.

The beneficial effects of the present invention are as follows: the air bag or balloon structure is adopted, and compared with a metal structure, the air bag has better protection for the human tissue against the traction part, and does not easily puncture the human tissue. At the same time, the catheter can be made more flexible, the outer wall of the catheter is thinner, and the human body is more comfortable during insertion. Because the air bag is made of a non-compliant or semi-compliant material, the shape of the air bag can be controlled by the air pressure, and the traction can be accurately and reliably achieved. Because use of the air bag structure is adopted, the diameter of the air bag is small, the flexibility is high, the air bag can enter a narrow cavity or a complex cavity, and meanwhile the comfort degree of the patient is improved.

### BRIEF DESCRIPTION OF THE DRAWINGS

FIG. 1 is a schematic structural view of a catheter portion according to the present invention.
FIG. 2 is a schematic view of a main shaft portion of the present invention.
FIG. 3 is a schematic view of the high-pressure braided tube and the two-way valve of the present invention.
FIG. 4 is a schematic view of the headband of the present invention.
FIG. 5 is a schematic view of a sheath tube of the present invention.
FIG. 6 is a schematic view of a three-way circular joint at the bottom of the sheath tube of the present invention.
FIG. 7 is a schematic diagram of the curved balloon catheter retractor after filling according to an embodiment of the present invention.

### DETAILED DESCRIPTION OF THE PREFERRED EMBODIMENTS

The present invention will be further described in detail below with reference to the accompanying drawings and embodiments.

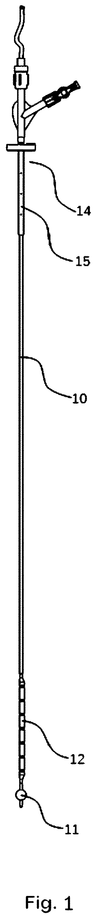

Referring first to FIG. 1, FIG. 1 is a schematic structural diagram of a catheter portion according to the present invention. As shown in FIG. 1, the distal end of the catheter 10 of the present invention has a positioning balloon 11, the catheter 10 has a curved balloon 12 near the distal end, and the balloon 11 and the curved balloon 12 are respectively connected to respective injection ports at the proximal end of the catheter by lumens inside the catheter 10.

The catheter 10 has a fixed handle 14 at the proximal end and a length scale portion 15 at a handle portion or handle 14.

In the unused state, the positioning balloon 11 and the curved balloon 12 are attached to the catheter 10 covered by and within a rigid tubular protective shield or tube 16.

In the use state, the balloon protective shield 16 is removed, and fluid is injected via the injection port of the positioning balloon 11, and the positioning balloon 11 is inflated to clamp or move the esophagus or tissue, so as to play a role in positioning. Fluid is then injected into the curved balloon 12 such that the curved balloon 12 and the catheter 10 therein are partially curved or bent and the esophagus is deviated, pulled or moved to one side.

The user may adjust the position of the curved balloon 12 through use of the handle 14 and may estimate the relative position of the curved balloon 12 by the length scale portion 15 of the handle 14.

The catheter 10 is made of a material that is not easily twisted, or may be a combination of a multi-lumen tube and a braided tube, or other combinations. When the part of catheter 10 left outside the human body cavity is rotated, the part of the catheter 10 within the cavity rotates at the same time by a certain proportion. At the same time, the catheter 10 has the length scale portion 15 for marking the depth of insertion of the catheter 10.

The fluid used here is typically liquid and gas. When the curved balloon 12 is inflated with fluid, it is bent toward one side. In one embodiment, the fluid is injected through the injection port by a syringe. Closing the catheter and valve after injection of the fluid may cause the curved balloon 12 to maintain fluid pressure. Generally, the curved balloon 12 is located outside the catheter 10, and the catheter portion where the positioning balloon 11 and the curved balloon 12 are located is provided with a radiopaque part so that its relative position can be displayed under X-rays. The radiopaque part may be a radiopaque material, such as tantalum, platinum iridium alloy, tungsten, etc. Other positioning devices, such as infrared or radio frequency tags, may also be used. In some embodiments, the catheter 10 is provided with a radiopaque line as a display under X-ray. There are three radiopaque parts within the curved balloon 12, located at the proximal, middle and distal ends of the curved balloon 12 respectively, which may display the relative position of the curved balloon 12, as well as the position of the largest curved or deviated portion.

In terms of the preferable length of the curved balloon 12, because the curved balloon 12 curves, moves or deviates the esophagus by forming a semicircle, the deviation distance relates to the radius of the semi-circle. That is, the longer the curved balloon 12 is, the more pronounced the deviation effect is. However, due to the configuration of the human esophagus, the distance between the second and third esophagus constriction or stenosis is from 11 cm to 15 cm, which is related to the person's height. When the curved balloon 12 is bent, if the curved balloon is located at the esophagus constriction, the curvature is limited at the esophagus constriction . Therefore the length of the balloon should satisfy the distance between the second and third constriction or stenosis of all people, and the longer the length of the balloon is, the better. Therefore, the length of the curved balloon 12 is preferably 9-11 cm.

The shape of the curved balloon 12 is segmented, but the entirety is internally connected or in communication; a single segment cannot constitute a curved balloon. Regarding the preferable number of segments of the curved balloon 12, the bending of curved balloon 12 is driven by the asymmetry of its shape and different degrees of expansion on both sides. The more segments there are, the increased asymmetry makes it easier for the curved balloon 12 to bend. If the curved balloon 12 bends into a semi-circle, it is the best. For two curved balloons 12 with different numbers of segments that are bent into the same semi-circle, a larger number of segments of the curved balloon 12 will make the diameter of the curved balloon 12 smaller, thereby reducing the stiffness of the curved balloon 12. The curved balloon 12 needs to meet a certain rigidity after being bent into a semicircle, and therefore the preferable number of segments of the curved balloon 12 is from 5 to 8.

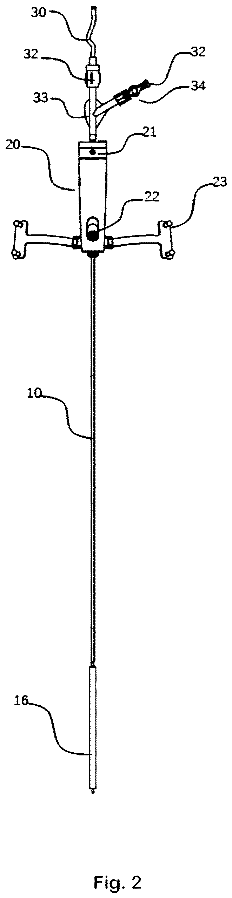

Please refer to FIG. 2, which is a schematic diagram of a main shaft portion or main shaft of the present invention in FIG. 2. The main shaft 20 is configured to hold or be held and to rotate.

A fixing screw 21 at the proximal end of the main shaft 20 can be used to clamp the catheter 10 passing through the main shaft 20. The distal part of the main shaft 20 is provided with a locking slider 22 for fixing or loosening the catheter 10 passing through the main shaft 20 to adjust the position of the catheter 10.

The distal portion of the main shaft 20 has two connecting buckles 23 for connecting a fixing headband so that the main shaft 20 and the entire catheter 10 are fixed relative to the head.

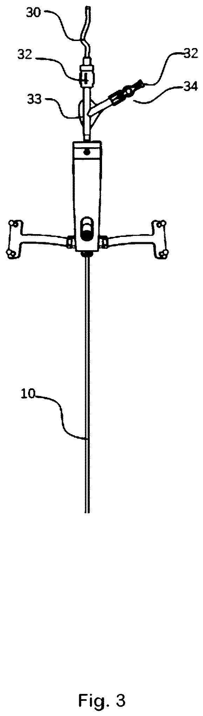

Referring to FIG. 3, which is a schematic diagram of a high-pressure braided tube and a two-way valve of the present invention.

One end of the high-pressure braided tube 30 is connected to the syringe 31, and another end of the high-pressure braided tube 30 is connected to the injection ports 32 of the curved balloon 12 or the positioning balloon 11. A switch 34 is provided in the middle of the two-way valve 33 and can be connected between the injection port 32 and the high-pressure braided tube 30 when needed for controlling fluid injection so as to maintain the pressure in the balloon.

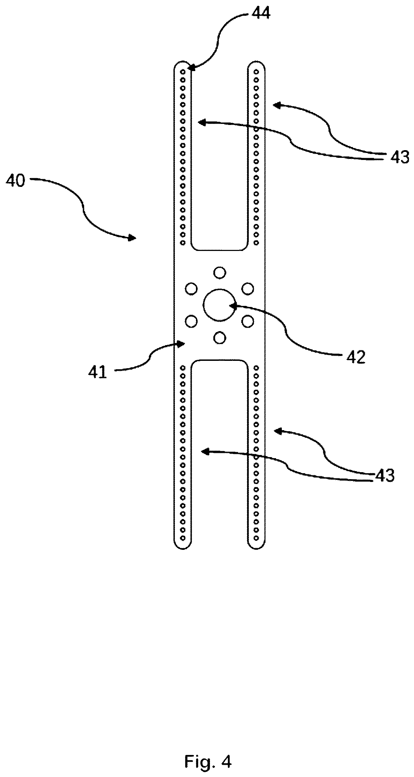

Please refer to FIG. 4, which is a schematic diagram of the headband of the present invention. The headband 40 is formed by a main body portion 41 to located at the back of a head, and the main body portion 41 is provided with a large hole 42 for accommodating a protruding portion behind or at the back of the head, making it more comfortable to wear. The main body portion 41 is provided with four connecting belts 43, and the connecting belt 43 is provided with a string of connecting holes 44 to clamp the two connecting buckles 23 at the head or head portion of the main shaft 20

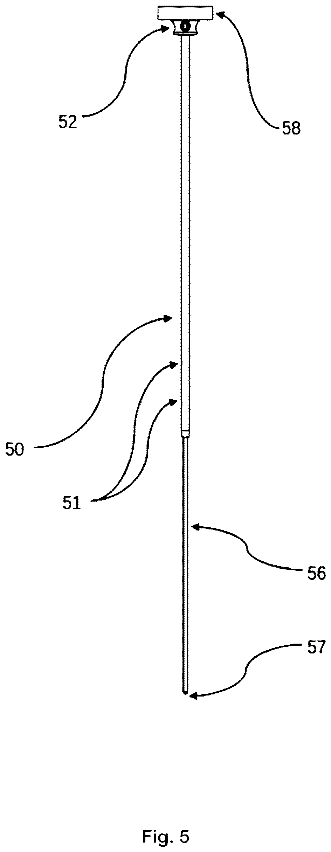

Please refer to FIG. 5, which is a schematic diagram of the sheath tube of the present invention. The sheath tube 50 is a hollow tube, and the tube is provided with a hole 51 for spraying a developing solution to display the position of the sheath tube. At the same time, because the catheter 10 needs to be rotated and pulled in the cavity, if such operation of the catheter 10 is performed in a human body cavity, such as the nasal cavity, it may cause a risk of scratching damage. However, the sheath tube 50 can form a fixed non-rotating protective portion, so that the upper portion of the human body cavity is not damaged by rotation and pulling of the catheter.

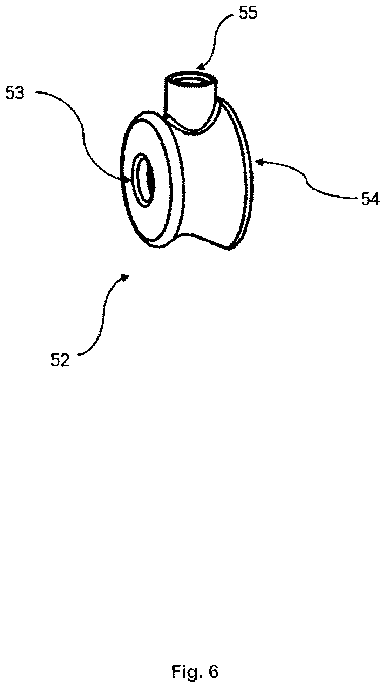

Please refer to FIG. 6, which is a schematic diagram of a three-way round joint at the proximal end of the sheath tube 50 of the present invention. The proximal end of the sheath tube 50 is provided with a three-way round joint 52, one end 53 of which is connected with the sheath tube, and another end 54 of which is connected with the main shaft 20 so as to accommodate the catheter 10. A side of the sheath tube 50 is provided with a hole 55 for injecting a developing solution.

The sheath tube auxiliary device 56 is a flexible solid tube having a smooth head 57 and an elliptical bottom 58.

The sheath tube auxiliary device 56 functions to smoothly enter the cavity by using the characteristics of the flexible solid tube, and simultaneously guide the sheath tube 50, so as to prevent the sheath tube 50 from being directly inserted into the cavity, such as the nasal mucosa or the esophageal wall, and to prevent the cavity from being scratched by the edge of the sheath tube. After the sheath tube 50 is introduced into the cavity by the sheath tube auxiliary device 56, the sheath tube auxiliary device can be pulled out smoothly. The catheter 10 is then inserted into the sheath tube 50.

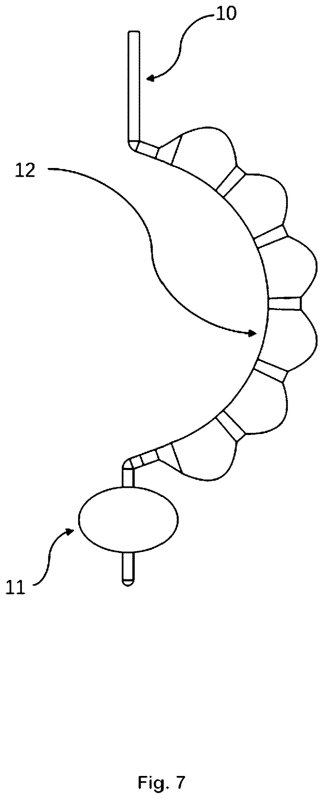

FIG. 7 is a schematic diagram of the curved balloon catheter retractor after inflation according to an embodiment of the present invention. In FIG. 7, the positioning balloon 11 and the curved balloon 12 at the distal end of the catheter 10 are in an inflated state.

The present invention also provides a retraction method, which can be expanded into an arc-shaped curved balloon 12 through a single-lumen/multi-lumen catheter, and which can be used to intervene or retract tissue in a human body. The curved balloon 12 is located outside the catheter 10. The catheter 10 can be offset towards the convex direction of the curved balloon 12 and can be provided with an operating handle 14 and other related accessories for use in combination. The said curved balloon catheter retractor can be used in surgeries for tissue retraction which is accomplished through interventional procedures or surgeries. Procedures or surgeries include, but are not limited to, various laparoscopic procedures, cardiovascular surgery, brain surgery, digestive tract surgery, urinary surgery, etc. The tissue to be retracted includes, but is not limited to, gastrointestinal tract, esophagus, trachea, urethra, vagina, bladder, etc. The purpose of the retraction include, but are not limited to, protecting a particular tissue, or removing a particular tissue to facilitate operation.

The catheter 10 of the curved balloon 12 of the retractor is controlled by the handle 14 during use to intervene with tissue within the body. With the help of the image device, the position and rotation angle of the catheter 10 can be adjusted with the handle 14. The positioning and angle can be locked with the set screw 21 on the main shaft 20. Inflation of the curved balloon 12 may be accomplished by the fluid injection port 32 to which the handle 14 is attached, or any other pathway. The curved balloon 12 expands and is bent after being filled with fluid, and at least one section of the lumen or balloon of the catheter 10 is bent to varying degrees, and finally the bent portion of the catheter can achieve the effect of moving, pulling or deviating the tissue.

The effect of the catheter retractor is described by taking atrial fibrillation ablation complications as an example. Atrial fibrillation is the most common arrhythmia, while atrial fibrillation radiofrequency ablation therapy has been gradually recognized in recent years as a better solution than drug therapy and conventional surgical procedures. The occurrence of atrial esophageal fistula complications arises from the spatial relationship between the left atrium and the esophagus. Since the esophagus is located in the posterior mediastinum and is only separated from the posterior wall of the left atrium by the pericardial sinus, the posterior wall of the left atrium and the anterior wall of the esophagus are both thin, and high temperature and high energy used during the ablation procedure are likely to cause excessive damage to the esophagus. The atrial esophageal fistula complications have extremely high mortality. In order to make the surgery more safe, the esophagus must be pulled away from the heart. With the present invention, the catheter 10 provided with the curved balloon 12 is inserted into the patient's body cavity, and the curved balloon 12 is partially inserted into the desired position within the tissue to be retracted, or a desired positioning effect. The catheter 10 is filled with fluid to inflate the balloon. The curved balloon 12 bends and pulls the catheter 10 to curve or bend and causes the patient's body cavity, such as the esophagus, to move or bend, thereby achieving the deviation displacement.

When in use, the catheter 10 is inserted into the esophagus from the nasal cavity, and when the positioning balloon 11 at the distal end enters between the esophagus second and third constrictions, the positioning balloon 11 at the distal end is inflated and pushed to the third constriction. Then the curved balloon 12 in the middle is expanded to bend or curve and drive or move the esophagus to deviate. During use, the catheter 10 and the balloon are driven to rotate by rotating the handle 14, so that the deviate direction of the esophagus changes, thereby avoiding the ablation point of the cardiac ablation all the time. The aperture may also be used to spray the developer such that the esophageal deviated portion and position is able to be seen or developed under x-rays.

Specifically, when the catheter is used for esophageal traction or retraction, the catheter is inserted into the esophagus through the nasal cavity or oral cavity first. After reaching the desired position, the relative position of the esophagus to the heart and bending or curving direction can be determined based on the markers 15 on the catheter 10 and the handle 14. Then the curved balloon 12 is inflated with fluid and is stiffened to bend, move or deviate the esophagus to a desired retraction position. When the position of the curved, bent or retraction balloon is rotated and adjusted on the desired horizontal section (when the ablation point is changed during cardiac ablation), the entire catheter 10 is rotated through the handle 14, and the curved balloon 12 is driven to rotate, thereby retracting the esophagus in a new direction to a new desired retraction position to make it stay away or keep it away from the new ablation point. The curved balloon 12 is located between the second and third constriction of the esophagus when the esophagus is deviated. The diameter of the positioning balloon 11 after expansion is greater than the diameter of the inner wall of the esophagus at the constriction. The length of the curved balloon 12 is 10-15 cm, the working air pressure is 2-8 atm, and the deviated displacement distance is 2-4 cm. Those skilled in the art can still make other modifications within the spirit of the present disclosure, and various modifications derived according to the spirit of the present disclosure shall fall within the scope of protection of the present disclosure.

## Claims

1. A curved balloon catheter retractor comprising: a distal end of a catheter is provided with a positioning balloon, close to the distal end of the catheter, and a bendable curved balloon having a segmented shape, and with the entire bendable curved balloon in internal fluid communication,
the positioning balloon and the bendable curved balloon are connected to respective injection ports at the proximal end of the catheter through lumens within the catheter,
a proximal end of the catheter is provided with a handle for adjusting the position of the catheter, and
wherein in the unused state, the positioning balloon and the bendable curved balloon are tightly attached to the catheter, and in the use state, a fluid is injected via the injection port of the positioning balloon to inflate the positioning balloon to achieve a desired positioning effect, and the fluid is injected into the bendable curved balloon to bend the bendable curved balloon and the catheter portion to a desired retraction position deviating to one side.

2. The curved balloon catheter retractor of claim 1, wherein, in the unused state, the positioning balloon and the bendable curved balloon are tightly attached to the catheter and covered by a rigid tubular protective shield, and the rigid tubular protective shield is removed in the use state.

3. The curved balloon catheter retractor of claim 1, wherein the handle has a length scale portion for marking the depth of the catheter insertion.

4. The curved balloon catheter retractor of claim 1, wherein the catheter is made of a material that is not easily twisted.

5. The curved balloon catheter retractor of claim 1, wherein the bendable curved balloon includes three radiopaque parts used for displaying the relative positions of the proximal end of the catheter, a middle part of the bendable curved balloon, and the distal end of the catheter, respectively.

6. The curved balloon catheter retractor of claim 1, wherein the length of the bendable curved balloon is preferably 9-11 cm.

7. The curved balloon catheter retractor of claim 1, wherein the number of curved balloon segments is in the range of 5 to 8.

8. The curved balloon catheter retractor of claim 1, wherein the proximal end of the catheter includes a main shaft for holding and rotating the catheter, and a proximal end of the main shaft includes a fixing screw for clamping the catheter passing through the main shaft, and a head portion of the main shaft includes a locking sliding block for fixing or loosening the catheter passing through main shaft for adjusting the position of the catheter and the bendable curved balloon.

9. The curved balloon catheter retractor of claim 1, wherein a high-pressure braided tube is secured at one end of the curved balloon catheter retractor and is interconnected with a syringe, and another end of the high-pressure braided tube is connected to an injection port of the curved balloon or positioning balloon at the distal end of the catheter.

10. The curved balloon catheter retractor of claim 9, wherein the catheter is further provided with a two-way valve having a switch in a middle portion, which can be connected between the injection port and the high-pressure braided tube when needed for controlling fluid injection and maintain pressure within the curved bendable balloon.

11. The curved balloon catheter retractor of claim 8, further provided with a fixing head band for a patient's head having a main body portion with a large hole for accommodating a protruding portion behind the patient's head, such that the protruding portion is more comfortable to wear.

12. The curved balloon catheter retractor of claim 11, wherein the main shaft has two connecting buckles for connecting the fixing headband such that the main shaft and the entire catheter are fixed relative to the patient's head.

13. The curved balloon catheter retractor of claim 11, **characterized in that** the fixing head belt main body is provided with four connecting belts, and the connecting belt is provided with a string of connecting holes which can clamp and connect with the two connecting buckles of the head portion of the main shaft.

14. The curved balloon catheter retractor of claim 1, further comprising a hollow sheath tube which is a fixed non-rotating protective portion.

15. The curved balloon catheter retractor of claim 14, wherein the hollow sheath tube includes a hole for spraying developing solution to display the position of the bendable curved balloon when in the use state.

16. The curved balloon catheter retractor of claim 14, wherein a radiopaque line is embedded within the hollow sheath tube and may be observed under X-ray.

17. The curved balloon catheter retractor of claim 14, wherein a bottom of the hollow sheath tube includes a three-way round joint, wherein one end of three-way round joint is connected with the hollow sheath tube, and another end of the three-way round joint is connected with a main shaft to accommodate the catheter, and a side edge of the three-way round joint is provided with a hole for injecting developing solution.

18. The curved balloon catheter retractor of claim 14, further including a sheath tube auxiliary device comprising a flexible solid tube having a smooth head and an oval bottom, wherein the sheath tube auxiliary device is a flexible solid tube to smoothly enter a human body cavity and guide a sheath tube positioned within the sheath tube auxiliary device to prevent damage by the sheath tube during insertion, and enable smooth removal prior to insertion of the catheter into the sheath tube in the use state.
